## Europäisches Patentamt

## European Patent Office

## Office européen des brevets

(19)

(11) Numéro de publication: **0 058 616**
**A1**

(12) **DEMANDE DE BREVET EUROPEEN**

(21) Numéro de dépôt: **82400250.5**

(22) Date de dépôt: **12.02.82**

(51) Int. Cl.³: **G 01 N 33/80**

(30) Priorité: **13.02.81 FR 8102866**

(43) Date de publication de la demande:
**25.08.82 Bulletin 82/34**

(84) Etats contractants désignés:
**BE CH DE GB IT LI NL SE**

(71) Demandeur: **Fella, Christian Paul**
**26, Boulevard de Cimiez**
**F-06000 Nice(FR)**

(71) Demandeur: **Hammou, Jean-Claude**
**32, Avenue du Dauphiné**
**F-06000 Nice(FR)**

(72) Inventeur: **Fella, Christian Paul**
**26, Boulevard de Cimiez**
**F-06000 Nice(FR)**

(72) Inventeur: **Hammou, Jean-Claude**
**32, Avenue du Dauphiné**
**F-06000 Nice(FR)**

(74) Mandataire: **Barnay, André François et al,**
**Cabinet Barnay 80 rue Saint-Lazare**
**F-75009 Paris(FR)**

(54) **Procédé de mise en évidence des antigènes de groupe sanguin A, B, H sur des cellules humaines en milieu liquide.**

(57) Pour mettre en évidence, fiablement et simplement, la présence ou l'absence d'antigènes de groupe sanguin, l'invention utilise un échantillon de cellules humaines en milieu liquide. On le maintient constamment sous pH de l'ordre de 7,3 en le soumettant à diverses étapes de centrifugation, exposition à un antisérum spécifique antigénique marqué par un agent fluorescent, et on soumet ensuite cet échantillon à une excitation de fluorescence. Il y a fluorescence si l'antisérum a été fixé sur un site antigénique.
Evaluation pronostique des tumeurs malignes.

EP 0 058 616 A1

Procédé de mise en évidence des antigènes de groupe sanguin
A,B,H sur des cellules humaines en milieu liquide.

La présente invention concerne un procédé de
mise en évidence des antigènes de groupe sanguin A,B,H
sur des cellules humaines en milieu liquide.

Ce procédé vise à mettre en évidence les antigènes de groupe sanguin A,B,H sur des cellules humaines
épithéliales recueillies soit dans les humeurs (urine,
liquide d'ascite, liquide pleural...), soit de cellules
recueillies après prélèvement par différents procédés :

- cellules d'un frottis vaginal mises en
suspension dans un milieu adéquat,

- cellules du col utérin mises en suspension dans
un milieu adéquat,

- cellules de lavage bronchique recueillies après
bronchoscopie,

- cellules d'expectoration mises en suspension dans
un milieu adéquat.

Le procédé selon l'invention se caractérise
par le fait qu'on place un prélèvement de cellules dans
une solution d'un milieu fixateur tel que l'éthanol dont
le pH est stabilisé entre 7 et 7,4 par un tampon approprié
(par exemple de type PBS), puis on centrifuge la suspension
cellulaire ainsi obtenue de façon à former un culot
cellulaire que l'on place ensuite dans un milieu comprenant
à la fois un tampon approprié à un pH de 7 à 7,4 et un
détergent non ionique connu en soi, de façon à former une
deuxième suspension cellulaire que l'on centrifuge pour
constituer un deuxième culot cellulaire,après quoi on
ajoute à ce deuxième culot cellulaire un antisérum spécifique humain d'au moins un antigène A, ou B ou O(H) marqué
par une subtance fluorescente telle que par exemple
fluorescéine ou rhodamine, après quoi on lave en milieu
tamponné à pH compris entre 7 et 7,4 pour éliminer l'antisérum non fixé et l'on effectue une excitation susceptible
de provoquer la fluorescence de la substance marquant
l'antisérum, et l'on décèle la présence ou l'absence de
fluorescence traduisant la fixation d'antisérum sur au moins un site antigénique.

L'excitation et la détection de la fluorescence peuvent être effectuées dans un appareil d'un type connu, appelé trieur-cellulaire ("cell sorter").

L'invention prévoit en outre que l'antisérum peut être monospécifique d'un seul antigène A ou B ou O(H).

Dans une variante, on peut opérer de façon telle qu'après addition de l'antisérum on ajoute une petite quantité de teinture histologique connue sous le nom de bleu Evans, on maintient un pH compris entre 7 et 7,4, on lave et l'on filtre, toujours sous le même pH, en retenant les particules supérieures à 5 $\mu$m que l'on place sur un montage microscopique , et que l'on examine sur microscope à fluorescence.

Dans une première phase, les cellules sont recueillies dans une solution comportant 50% d'éthanol qui sert de milieu fixateur avec un milieu tampon de type PBS (tampon phosphate) représentant les 50% restants.

Dans une deuxième phase, on centrifuge la suspension cellulaire ainsi obtenue (par exemple 5 minutes à 5000 tours minute). On récupère le culot cellulaire dans un milieu comprenant un tampon phosphate de type PBS (90%) associé à un détergent non ionique (10%), par exemple un produit commercialisé sous le nom de TWEEN fait parfaitement l'affaire.

Dans une troisième phase, on centrifuge à nouveau la suspension cellulaire ainsi obtenue. On récupère le culot dans lequel on ajoute à quantité égale au culot l'antisérum marqué. Il s'agit d'un antisérum marqué par une substance fluorescente (fluorescéine ou rhodamine par exemple).

La spécificité de ce sérum sera dirigée soit contre l'antigène A de groupe sanguin, soit contre l'antigène B, soit contre l'antigène A et l'antigène B dans le même sérum, soit contre l'antigène H de groupe sanguin. Si les cellules du culot cellulaire recueillies chez le patient sont du groupe A par exemple, l'antisérum utilisé sera un antisérum de spécificité anti A. Il en est de même pour les autres groupes sanguins B, AB, O.

Il existe une possibilité de rechercher l'antigène de groupe sanguin A, B, AB, O à la surface des cellules dans la préparation définie plus haut sans connaître le groupe sanguin du patient d'où proviennent les cellules. En effet, on peut réaliser un sérum polyvalent ayant plusieurs spécificités en mélangeant à part égale chaque antisérum (de spécificité anti A, anti B, de spécificité anti H). On a donc ainsi réuni dans une seule préparation plusieurs antisérums recouvrant toutes les spécificités de groupe sanguin A, B ou O(H).

Il existe deux possiblités de lecture suivant les moyens mis en oeuvre: soit lecture en microscopie optique avec un microscope à fluorescence, soit lecture par un appareil de type "cell sorter" (appareil automatique à analyser et à trier les cellules).

Dans le cas où l'on veut réaliser une lecture par microscope optique par fluorescence, on ajoute à l'antisérum utilisé quel qu'il soit, soit un antisérum monospécifique (ayant soit la spécificité anti A, soit la spécificité anti B, soit la spécificité anti H), soit un sérum polyspécifique ayant donc plusieurs spécificités (anti A + anti B + anti H).

On rajoute à cet antisérum un colorant, le bleu Evans, après quoi on fait une centrifugation qui servira de lavage dans un tampon de type PBS. On remet ensuite en suspension dans 5 cm$^3$ d'un tampon phosphate (PBS) dans une seringue et on filtre sur millipore. On fait ensuite un montage du millipore en glycérol sur coupe histologique. On fait ensuite une lecture en miscroscope à fluorescence.

La deuxième possibilité de lecture est celle donnée par un appareil de type "cell sorter" (appareil automatique connu pour analyser et trier les cellules), après application de l'antisérum marqué soit monospécifique (anti A ou anti B, ou anti H), soit polyspécifique (anti A + anti B + anti H). Dans ce cas, il est avantageux d'utiliser un antisérum polyvalent contenant plusieurs antisérums.

La suspension cellulaire réalisée plus haut est alors centrifugée dans un tampon phosphate de type PBS. Le culot cellulaire recueilli est à nouveau mis en suspension dans un tampon de type PBS et l'échantillonnage est passé à la machine "cell sorter".

Il existe une possibilité de double marquage des cellules en suspension. En effet, au temps d'application de l'antisérum marqué sur les cellules, la suspension cellulaire peut être séparée en deux fractions avant le contact avec l'antisérum.

La première fraction est mise en contact avec l'antisérum soit monospécifique, soit polyspécifique qui peut être marqué par une substance fluorescente de type fluorescéine. La deuxième fraction est mise en contact avec un antisérum marqué qui n'a pas la spécificité A, B ou H mais une autre spécificité (par exemple un antisérum dirigé contre un autre antigène de la membrane cellulaire); cet antisérum peut être également dirigé contre un antigène se trouvant dans le cytoplasme (ou sur le noyau de la cellule).

Cet antisérum marquant la deuxième fraction cellulaire sera marqué avec une substance fluorescente différente, par exemple la rhodamine. La lecture pourra se faire soit avec le microscope optique à fluorescence, soit avec une machine de type "cell sorter".

REVENDICATIONS

1.- Procédé de mise en évidence des antigènes de groupe sanguin A, B, H sur des cellules humaines en milieu liquide, caractérisé par le fait qu'on place un prélèvement de cellules dans une solution d'un milieu fixateur tel que l'éthanol dont le pH est stabilisé entre 7 et 7,4 par un tampon approprié (par exemple de type PBS), puis on centrifuge la suspension cellulaire ainsi obtenue de façon à former un culot cellulaire que l'on place ensuite dans un milieu comprenant à la fois un tampon approprié à un pH de 7 à 7,4 et un détergent non ionique connu en soi, de façon à former une deuxième suspension cellulaire que l'on centrifuge pour constituer un deuxième culot cellulaire après quoi on ajoute à ce deuxième culot cellulaire un antisérum spéficique humain d'au moins un antigène A, ou B ou O(H) marqué par une substance fluorescente telle que par exemple fluorescéine ou rhodamine, après quoi on lave en milieu tamponné à pH compris entre 7 et 7,4 pour éliminer l'antisérum non fixé et l'on effectue une excitation susceptible de provoquer la fluorescence de la substance marquant l'antisérum, et l'on décèle la présence ou l'absence de fluorescence traduisant la fixation d'antisérum sur au moins un site antigénique.

2.- Procédé selon la revendication 1, caractérisé par le fait que l'excitation et la détection de la fluorescence sont effectuées dans un appareil d'un type connu, appelé trieur-cellulaire ("cell sorter").

3.- Procédé selon l'une quelconque des revendications 1 ou 2, caractérisé par le fait que l'antisérum est monospécifique d'un seul antigène A ou B ou O(H).

4.- Procédé selon l'une quelconque des revendications 1 à 3, caractérisé par le fait qu'après addition de l'antisérum on ajoute une petite quantité de teinture histologique connue sous le nom de bleu Evans, on maintient un pH compris entre 7 et 7,4 , on lave et l'on filtre, toujours sous le même pH , en retenant les particules supé-rieures à 5 $\mu$m que l'on place sur un montage microscopique, et que l'on examine sur microscope à fluorescence.

**0058616**

Office européen
des brevets

**RAPPORT DE RECHERCHE EUROPEENNE**

Numéro de la demande

EP 82 40 0250

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | FR-A-1 602 301 (STERWIN A.G.) <br> * revendications 1,2 * <br><br> --- | 1,4 | G 01 N 33/80 |
| Y | CHEMICAL ABSTRACTS, vol.79, no.1, 9 juillet 1973, résumé no. 3648j, page 326, Columbus, Ohio (US), &Z. Immunitaetsforsch., Exp. Klin. Immunol, 1972, 144(5), 482-90, H. SCHULTZE-MOSGAU et al.:"Detection of specific blood-group-receptors in the placenta with agglutinins from plants (lectins) and snail s (protectins)". * abrégé en entier * <br><br> --- | 1 | |
| X | SCIENTIFIC AMERICAN, vol.234, no.3, mars 1976, New York (US), L.A. HERZENGERG et al.:"Fluorescence-activated cell sorting", pages 108-117 * article en entier * <br><br> --- | 2 | |
| Y | US-A-4 172 227 (BECTON, DICKINSON AND COMP.) * en entier * <br><br> ----- | 2 | |

**DOMAINES TECHNIQUES RECHERCHES (Int. Cl. 3)**

G 01 N

---

Le présent rapport de recherche a été établi pour toutes les revendications

| Lieu de la recherche <br> LA HAYE | Date d'achèvement de la recherche <br> 24-05-1982 | Examinateur <br> MEYLAERTS H. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503. 03.82